Europäisches Patentamt

European Patent Office

Office européen des brevets

(19)

(11) Publication number: **0 363 792 B1**

# EUROPEAN PATENT SPECIFICATION

(45) Date of publication of patent specification: **18.01.95**

(51) Int. Cl.6: **C12P 17/16**, C12P 1/00, C12N 15/53

(21) Application number: **89118346.9**

(22) Date of filing: **03.10.89**

(54) **Melanin production.**

(30) Priority: **03.10.88 US 251809**

(43) Date of publication of application:
**18.04.90 Bulletin 90/16**

(45) Publication of the grant of the patent:
**18.01.95 Bulletin 95/03**

(84) Designated Contracting States:
**AT BE CH DE ES FR GB GR IT LI LU NL SE**

(56) References cited:
**WO-A-88/02372**

**CHEMICAL ABSTRACTS, vol. 98, no. 1, 3rd January 1983, page 325, abstract no.3561t, Columbus, Ohio, US; & PL-A-114 875 (Akademia Rolnicza, Krakow) 30-09-1982**

**CHEMICAL ABSTRACTS, vol. 108, no. 15, 11th April 1988, page 417, abstract no.128189j, Columbus, Ohio, US; H. PLATEN et al.: "Effect of copper on growth andtyrosinase activity of streptomycetes", & VDLUFA-Schriftenr. 1987,**

**20(Leistungsfoerderer Tierprod.), 859-69**

(73) Proprietor: **BIOSOURCE GENETICS CORPORATION**
**3333 Vaca Valley Parkway**
**Vacaville, CA 95688 (US)**

(72) Inventor: **Grill, Laurence K.**
**3570 Cantelow Road**
**Vacaville, California 95688 (US)**
Inventor: **Garger, Stephen J., Jr.**
**593 Cottonwood Street**
**Vacaville, California 95688 (US)**
Inventor: **Sverlow, Genadie D.**
**851 Malibu Drive**
**Concord, California 94518 (US)**
Inventor: **Erwin, Robert L.**
**336 Summerfield Drive**
**Vacaville, California 95687 (US)**

(74) Representative: **Müller-Boré & Partner Patentanwälte**
**Postfach 26 02 47**
**D-80059 München (DE)**

CHEMICAL ABSTRACTS, vol. 95, no. 19, 19th November 1981, page 379, abstract no.165240c, Columbus, Ohio, US; G.V. PAV-LENKO et al.: "Melanin pigment of-Gluconobacter oxydans", & MIKROBIOLOGIYA 1981, 50(4),718-22

CHEMICAL ABSTRACTS, vol. 89, no. 15, 9th October 1978, page 294, abstract no.125900n, Columbus, Ohio, US; F. GULYAS: "Studies of pigment formation byActinomycetes", & SOIL BIOL. CONSERV.BIOSPHERE, [PROC. MEET.], 7th 1975 (Pub. 1977), 265-70

CHEMICAL ABSTRACTS, vol. 72, no. 1, 5th January 1970, page 78, abstract no.878d, Co-lumbus, Ohio, US; S.J. PIRT et al.: "Melanin production in Aspergillusnidulans", & BIOCHEM. J. 1969, 114(1), 9P-10P

TRANS. MYCOL. SOC. Vol 70(3) 1978 p 453-455 B.I. ROWLEY et al: "Influence ofg-rowth rate history on production of melanin by Aspergillus Nidulans"

JOURNAL OF GENERAL MICROBIOLOGY, Vol 129, 1983, p 2703-2714 E. KATZ et al:"Cloning and Expression of the tyrosinase gene from Streptomyces antibioticusin Streptomyces lividans"

J. Clin. Microbiol., Vol. 10 (5), 1979, pp. 724-729

## Description

Melanins are dark-brown pigments found in animals, plants, and microorganisms. They are generally considered to enhance survival of the organism. Melanins have been associated with disease resistance in plants, Bell, A.A. et al., *Can. J. Microbiol.* 22, 787 (1976) and with immunity to parasites in invertebrate animals, Poinar, G.O. Jr., in *Immunity to Parasitic Animals*, eds. G.J. Jackson et al., p. 173, Appleton-Century-Croft, New York (1969). Melanins are associated with skin disorders, Breathnach, A.S., in *Pigments in Pathology*, p. 353, Academic Press, New york (1969), and malignant melanomas, Liban, E., in *Pigments in Pathology* p. 421, Academic Press, New York (1969).

The study of melanins has led to the discovery of a number of pathways of biosynthesis and also to a wide variety of chemicals related to melanin. Fungal melanin occurs as wall-bound melanin and extracellular melanin. Most hyphal, conidial, and sclerotial walls of melanized fungi appear to have two distinct layers: an inner layer which is electron translucent and an outer layer containing electron-dense granules. Collective evidence shows that these granules are melanins, Wheeler, M.H. et al., *Exp. Mycol.* 3, 340 (1979). Extracellular melanins are synthesized apart from cell walls. They are derived from phenols by two mechanisms: (a) oxidation of phenolic compounds by phenol oxidases (sometimes also called phenyloxidase) secreted into the medium and (b) oxidation of phenols secreted into the medium either by autooxidation or by enzymes released during autolysis. Wheeler, M.H. et al., *Can J. Microbiol.* 24, 289 (1978).

Fungi or bacteria which secrete tyrosinase cause discoloration of the surrounding medium. That discoloration can be accentuated by adding tyrosine to the medium, Hollis, J.P., *Phytopathology 42,* 273 (1952); Nurudeen, T.A. et al., *J. Clin. Microbiol. 10,* 724 (1979). Extracellular melanin has been observed in Actinomycetes, bacteria, and fungi. Genes controlling extracellular tyrosinase production or secretion occur on plasmids in *Streptomyces scabies,* Gregory, K.F. et al., *J. Bacteriol. 87,* 1287 (1964), and *Rhizobium phaseoli* strain 1233, Beynon, J.L. et al., *J. Gen. Microbiol. 120,* 421 (1980). The tyrosinase gene in *Vibrio cholerae* is located on the chromosome. Bell, A.A. et al., *Ann. Rev. Phytopathol. 24,* 411 (1986).

Fungal melanin is derived from tyrosine, $\gamma$-glutaminyl-3,4-dihydroxybenzene, or catechol. In one of the melanin pathways synthesis is mediated by a copper containing enzyme tyrosinase [EC 1.14.18.1] which is generally agreed to catalyze the first two steps in the biosynthesis. The initial reaction involves the hydroxylation of tyrosine. An oxygen atom is incorporated adjacent to the hydroxyl group of tyrosine to produce 3,4-dihydroxyphenylalanine (DOPA). Tyrosinase then catalyses the removal of two hydrogens to yield DOPA semiquinone then dopaquinone. The dopaquinone formed is not stable at physiological pH. The amino group of the side chain adds on intramolecularly to give leucodopachrome which is then oxidized rapidly to dopachrome, a red compound. The next step is a rearrangement and decarboxylation to give 5,6-dihydroxyindole (DHI) or without decarboxylation to produce 5,6-dihydroxyindole-2-carboxylic acid (DHICA). The eumelanins are formed from the polymerization of dopaquinone, dopachrome, indole-5,6-quinone, melanochrome or combinations thereof. These form the brown pigments in animals. Mason, H.S., *J. Biol. Chem. 172,* 83 (1948); and Pawelek, J.M. et al., *Am. Sci. 70,* 136 (1982). Phaeomelanins, the red, brown and yellow pigments of animals are polymers of cysteinyl DOPA or mixed polymers of DOPA and cysteinyl DOPA. Fitzpatrick, T.B. et al., in *Biology and Diseases of Dermal Pigmentation* p. 3, Univ. Tokyo Press, Tokyo. Allomelanins are formed from nitrogen-free precursors, primarily catechol and 1,8 dihydroxynaphthalene. Trichochromes are also classified with melanins since they are yellow, red and violet pigments and they are derived from the oxidation of tyrosine. Tyrosinase is not the only melanin producing enzyme. Laccase, an enzyme found in the outer walls of fungi is responsible for the oxidation of DOPA. it will not readily oxidize tyrosine. Simon, L.T. et al., *J. Bacteriol. 137,* 537 (1979). Other enzymes present in pigment producing organisms are phenyloxidase of *Cryptococcus neoformans* as well as catechol oxidase and other polyphenol oxidases of plants (Mayer, A.M. et al., *Phytochem. 18,* 193 (1979)).

$\gamma$-glutaminyl-3,4-hydroxybenzene (GDHB) melanin is synthesized from $\gamma$-glutaminyl-4-hydroxybenzene (GHB) by that action of tyrosinase in *Agaricus bisporus.* Hegnauer, H. et al., *Exp. Mycol. 9,* 221. Melanin is formed by non-enzymatic polymerization.

Catechol melanin is formed by the combination of catechol with semiquinone radicals to form catechol dimers and certain fused hetero-ring derivatives which then polymerize to form melanin. *Ustilago maydis* is believed to metabolize catechol to melanin. Teleospores of *U. maydis* produce highly election dense melanins when fixed with $OsO_4$. Patgieter, H.J. et al., *J. Bacteriol. 91,* 1526 (1966).

Biosynthesis of 1,8-dihydroxynaphthalene (DHN) melanin is produced from pentaketide. A variety of intermediates occur including 1,3,6,8-tetrahydroxynaphathlene, scytalone, 1,3,8-trihydroxylnaphthalene, vermelone, dihydroxynaphthalene, dihydroxynaphthalene 1,1-dimer and dihydroxynaphthalene 2,2-dimer. Mutational blocks eliminating reductase or dehydratase enzymes, and enzyme inhibitors such as tricyclazole cause the occurrence of a large number of shunt products. Wheeler, M.H. et al., *Arch. Microbiol. 142,* 234 (1985); and Stipanovic, R.D. et al., *Pestic. Biochem. Physiol. 13,* 198 (1980).

Culture conditions vary among different microorganisms. Extracellular melanin production in some microorganisms has been shown to increase as the concentration of tyrosine is increased to its saturation point of 0.1 percent. This percentage is considered supersaturation in tyrosine. Hollis, J.P. (1952), *supra.* It has been reported that yeast autolysates and casein hydrolysate stimulate melanin pigment production by *Streptomyces scabies* in a medium containing 0.1% percent tyrosine. Hollis, J.P., *supra.*

It has also been shown that melanin production is repressed by a variety of carbon sources. The particular carbon source varies with the microorganism. Nurudeen, T.A. et al. (1979), *supra,* reported that increased glucose concentration in the medium reduced pigmentation of all serotypes of *Cryptococcus neoformans.* This fungus produces melanin-like pigments with diphenol and aminophenol through the mediation of a phenyloxidase enzyme. The phenyloxidase of *C. neoformans* cannot use tyrosine as a substrate. In contrast to the metabolism of *Cryptococcus, Gluconobacter oxydans,* a pigment producing bacterium produces melanin in the presence of glucose and tyrosine, but not in a medium containing sucrose, fructose, sorbitol, mannitol or glycerol as the carbon source. Pavlenko, G.V. et al., *Microbiology USSR 50,* 539 (1981).

Several fungi are known to produce extracellular heterogenous melanins. These melanins are derived from various phenols, amino acids, proteins, carbohydrates and lipids. Synthesis requires secretion of phenols into the medium. Melanization occurs at alkaline pH.

In Chemical Abstracts, vol. 72, (1970), page 78, abstract 878d, the melanin production in aspergillus nidulans is described. The cloning and expression of the tyrosinase gene from Streptomyces antibioticus in Streptomyces lividans is described (Katz E., Jornal of General Microbiology, (1983), 129, 2703-2714).

The present invention is directed to processes for producing melanins, their precursors and their derivatives, hereinafter referred to generically as melanins. According to the invention, melanins are produced in amounts greater than about 0.2 grams dry weight per liter of growth medium. The enhanced production of melanin can be achieved by manipulating the constituents of the growth medium, and/or attenuating fermentations conditions and/or by genetically engineering microorganisms to produce melanins. Melanin producing microorganisms will generally proliferate in a variety of media known in the art for the microorganism from which it was derived. However a growth medium may be enhanced by the addition of special factors in order to increase the yield of melanins or to direct the yield of melanin precursors or derivatives and/or by the deletion of factors which negatively affect the yield of melanins.

Furthermore, the present invention relates to a culture comprising a growth medium, a microorganism capable of producing melanin and at least 0.2g dry weight of melanin per liter.

Suitable microorganisms are produced by mutagenesis and/or transformation by a number of methods conventional in the art. Mutagenesis is carried out by a number of methods which include, for example, radiation and exposure to mutagenic chemicals. Plasmids which contain genes coding for enzymes which lead to melanin production and an appropriate promoter for expression in the desired host are used to transform microorganisms which either do not produce melanin or produce melanin in commercially unsatisfactory amounts.

The present invention is directed to processes for producing melanins, their precursors and their derivatives, hereinafter referred to generically an melanins. According to the invention, melanins are produced in amounts greater than about 0.2 grams dry weight per liter of growth medium. The enhanced production of melanin can be achieved by manipulating the constituents of the growth medium, and/or attenuating fermentations conditions and/or by genetically engineering microorganisms to produce melanins. The present invention includes: (a) vectors capable of expressibly transforming microorganisms to produce microorganisms with the capability or enhanced capability of producing melanins; (b) unique microorganisms with the capability or enhanced capability of producing melanins; (c) a process of producing melanins with a microorganism; (d) a unique method of isolating melanins from cultures of microorganisms; and (e) a growth medium in which melanins can be produced in quantities greater than about 0.2 grams dry weight of melanins per liter of growth medium.

In order to provide a clear and consistent understanding of the specification and the claims, including the scope given to such terms, the following definitions are provided:

Melanin: A high molecular weight amorphous polymer of indole quinone including eumelanins, phaeomelanins, neuromelanins, allomelanins and trichochromes.

Microorganisms that produce melanins or other pigments are widely distributed in nature. Examples of such microorganisms include but are not limited to the following group of microorganisms, which however are preferred for the claimed method of producing melanins; Streptomyces, Rhizobium, Agaricus, Ustilago, Cryptococcus, Gluconobacter, Pseudomonas, Xanthomonas, Cochliobolus, Pleospora, Alternaria, Aurobasidium, Botrytis, Cladosporium, Diplodia, Sclerotium, Verticillium, Eurotium, Aspergillus, Stachybotrys, Hendersonula, Streptoverticillium, Micromonospora, Escherichia Bacillus, Streptococcus, Salmonella, Staphylococcus and Vibrio.

Several microorganisms may be genetically engineered to enable them to produce melanins. They include but are not limited to *Streptomyces, Escherichia, Bacillus, Streptococcus, Salmonella, Staphylococcus,* and *Vibrio.*

Industrial pigment production in microorganisms is preferred because extracellular melanins and their derivatives and precursors are easily extracted from the medium into which they have been excreted.

Microorganisms that produce melanins or similar pigments can be enhanced by alteration. Common alterations include plasmid insertion and mutation. Mutation is accomplished by a variety of means conventional in the art. Microorganisms can be exposed to ultraviolet or gamma radiation or mutagenic chemicals.

As previously described, the present invention is directed to the enhanced production of melanin in amounts greater than about 0.2 grams, especially in amounts greater than about 0.5 grams, preferably in amounts greater than about 1.0 grams and most preferably in amounts gerater than about 2.0 grams by weight per liter of medium. The enhancement can be achieved by the growth conditions of the microorganisms, such as medium constituents or attenuating fermentation and/or by genetic manipulations.

A. Growth Conditions

The basic composition of the medium used depends upon a number of considerations. The medium is usually chosen from those known in the art for growing microorganisms similar to the one being grown for melanin production. A variety of carbon and nitrogen sources are available and interchangeable. To enhance growth, the specific needs of the microorganism are considered and met. For instance, a microorganism may require a specific amino acid to be present. All microorganisms require metal ions such as iron and manganese but differ in their concentration requirements. Microorganisms are inhibited by the presence of certain metabolites. Inhibition differs widely from one microorganism to the next.

In addition to the metabolic needs of the microorganism the substrates available for enhancing melanin production should be considered. The presence of precursors may enhance the production or alter the composition of the melanin produced and thereby alter its color and its molecular weight or it may result in production of other desirable products. Tyrosinase requires copper. Therefore trace metals must be present in sufficient concentration to act as a cofactors for the enzyme without poisoning the medium.

The temperature of the fermentation medium is critical to optimum growth of the microorganism. Typical soil microorganisms grow well at about 26°C to about 30°C while *E. coli* grows best at 37°C and thermophilic microorganisms grow well at 50-60°C.

The pH of the medium is usually maintained between 6.6 and 7.2. A buffering agent which also provides a requirement of the microorganism is often chosen. A phosphate buffer is often used to maintain the pH of the medium.

Oxidative microorganisms require aeration. In smaller vessels, stirring or shaking is sufficient. In larger fermentation vessels oxygen is sparged into the system and an impeller stirs the medium at a rate sufficient to provide an optimum dissolved oxygen level. This might be around 20-90% for some microorganisms.

One means of enhancing melanin production is by modification of the growth medium. Applicants have found that several factors can be altered which tremendously increase melanin production. One factor is to attenuate the fermentation conditions. This is accomplished by maintaining a high level of oxygen availablility to the cultured microorganisms. If low levels of oxygen are available or a low degree of aeration is provided, then yields of melanin are reduced. A second factor is the presence of suitable tyrosine containing substrates such as casein hydrolysate or casein peptone. It has been found that either of these substrates are better than casamino acids. A third factor is the presence of tyrosine as an inducer. It has been found that the best yields occur at a tyrosine concentration of 1.5 g/l. A fourth factor is to delete from the medium glucose which act as repressors.

In general, cultures of microorganisms are grown in the selected medium described above to produce the desired level of melanin. The growth of the cultures and melanin production is observed by measuring the optical density at 400nm ($OD_{400}$) at various intervals of time. The $OD_{400}$ of 0.8 is indicative of a yield of 0.5 g/l. The $OD_{400}$ is monitored and the cultures are harvested when it has topped off. This usually occurs at an $OD_{400}$ of about 1.52 in shaker flasks and about 1.3 in a bioreactor.

The present inventor have found that by modifying their medium in which *S. lividans* TK64 (pIJ702) is grown a tremendous increase in melanin production can be achieved. By inventing a medium that lacks glucose, it has been found advantageously that melanin production can be increased in a fixed volume bioreactor from about 100 mg per liter to about 5.0 grams per liter or more.

The present invention includes a growth medium having a nitrogen source rich in tyrosine. It is also lacking in glucose. It has been found that higher melanin production is achieved with casein peptone than with casein hydrolysate or casaminoacids. It has also been found that tyrosine production is enhanced in *Streptomyces lividans* by the removal of glucose from the medium. There are at least two possible explanations for this effect, although applicants are not bound by those explanations. Glucose might be a preferred carbon and energy source for *S. lividans*. Its preferred biochemical pathway would then utilize all of the glucose in the medium before the pathway which utilizes tyrosine as a substrate is used. Another possible explanation is that glucose is a metabolic inhibitor of tyrosinase or some other enzyme in the biochemical pathway for melanin production.

It has been found that on inoculum of about $10^3$ to about $10^7$, prefereably about $10^4$ to about $10^6$, spores per ml produce high melanin yields. A starter culture that is in mid-log phase, about 24-48 hours old is typically added to the fermentation vessel at up to 10% volume.

B. Genetic Manipulations

The chimeric genes and vectors used in the present invention are constructed and used to transform microorganisms using techniques well known in the art in view of the following description. Suitable techniques have been described in Maniatis, T. et al., *Molecular Cloning*, 2nd Ed., Cold Spring harbor Laboratory, New York (1982); *Methods in Enzymology*, Vols. 68 (1979), 100 (1983), 101 (1983), 118 (1986) and Vols. 152-154 (1987); *DNA Cloning,* Glover, D.M., Ed., IRL Press, Oxford (1985); and *Plant Molecular Biology: Manual,* Gelvin, S.B. et al., Eds., Kluwer Academic Publishers, Dodrecht (1988). Medium compositions have been described in Miller, J.H., *Experiments in Molecular Genetics,* Cold Spring Harbor Laboratory, New York (1972), as well as the references previously identified.

Preparation of DNA Sequences

A DNA fragment from any source can be isolated and prepared by any of a variety of methods that are conventional in the art. Restriction endonucleases are useful because they allow DNA to be dissected, analyzed and restructured in a controlled, site-specific and predictable manner. A DNA sequence obtained from digestion by, for example, *Eco*RI will fit into an opening at a *Eco*RI recognition site in the plasmid DNA. The complementary ("sticky") ends are attachable by a T4 DNA ligase.

Digested DNA can be resolved on the basis of molecular weight by polyacrylamide or agarose gel electrophoresis. DNA fragments separated on an electrophoresis gel can be identified by a number of methods known in the art. They may be bound to protein or RNA and identified by electrophoresis. They may be probed by RNA or DNA after being lifted from a gel to cellulose nitrate filter paper. The RNA or DNA probe either carries a radioactive species or a detectable enzyme. Smith, G. et al., *Anal. Biochem. 109,* 123 (1980); and Southern, E.M. *J. Mol. Biol. 98,* 503 (1975). Synthetic oligonucleotide DNA probes are usually about 12 or more bases in length. If the sequence of the DNA or RNA sample is known, an exact probe can be synthesized. Lathe, R., *J. Mol. Biol. 183,* 1 (1985). A $^{32}$P-labeled DNA probe generated by nick translation can be hybridized to DNA fragments separated on an agarose gel and blotted onto cellulose nitrate. This process is known as southern blot hybridization. Wahl, G. et al., *Proc. Natl. Acad. Sci.*, USA 76, 3683 (1979).

The tyrosinase gene has been cloned from *Streptomyces antibioticus* DNA by digestion with *Bcl*I. DNA fragments were ligated to *Bcl*I - cleaved pIJ37 or to BamHI-digested pIJ41. Ligation mixtures were then used to transform protoplasts of *Streptomyces lividans* 1326. Katz, E. et al., *J. Gen. Microbiol*. 129, 2703 (1983). In addition, a tyrosinase gene can be isolated from any organism which produces melanin. Thus, the gene can be isolated from human hair, melanocytes or melanomas, cuttlefish, and red roosters, among others.

Transformation Vectors

The vectors used in the present invention are vectors which contain DNA coding for enzymes which catalyzes the production of melanin, its precursors and its derivatives. The DNA may be native to the intended host or foreign DNA. Foreign DNA is DNA which is exogenous to or not naturally found in the organism to be transformed. It can be inserted into cloning vectors to transform a host organism. The foreign DNA of the present invention is derived from or has substantial sequence homology to DNA of organisms which naturally produce melanin. The vectors of the present invention are produced by standard techniques. Appropriate vectors which can be utilized as starting materials are known in the art.

The construction of the vectors can be performed in a suitable host, for example, *E. coli*. A DNA sequence coding for enzymes which catalyse the formation of melanin, its precursors and its derivatives is obtained by conventional means and inserted into any vector suitable for the transformation of microorganisms. For example, the DNA sequence can be isolated from a gene bank of genomic clones. Alternatively, the DNA sequence can be prepared by reverse transcription. The vectors are then introduced into host cells by a variety of known techniques which give rise to a transformed host.

The DNA sequence can be chemically synthesized if the amino acid sequence of the enzymes which catalyze the production of melanin or part thereof is known. Several prior art methods can be utilized to determine the amino acid sequence of the enzymes. A part of the amino acid sequence can be determined and used to prepare a primer for reverse transcriptions. The DNA sequence can contain a coding sequence for the specific amino acid sequence of the enzyme. Alternatively, the DNA sequence can contain additional coding sequences which code for all or part of the enzyme. For example, the DNA sequence could code for the entire amino acid sequence of tyrosinase or it could code for a substantial portion of the amino acid sequence of tyrosinase.

The DNA sequence coding for the enzyme or part thereof is inserted into an appropriate vector in such a manner that the enzyme is correctly expressed. In other words, the DNA sequence is positioned in the proper orientation and reading frame so that the correct amino acid sequence is produced upon expression of the DNA sequence in the host. In accordance with conventional techniques, a chimeric DNA sequence is generally constructed which contains a promoter operable in the specific host microorganism and the DNA sequence coding for the desired enzyme. The chimeric DNA sequence may further contain 3'non-coding sequences operable in the host. The chimeric DNA sequence can be prepared *in situ* within a suitable vector by inserting the DNA sequence coding for the enzyme into a restriction site of a known host transformation vector. Alternatively, the chimeric gene could be first constructed and then inserted into a vector to produce a transformation vector. The vector can be further modified by utilizing an enhancer sequence and/or a strong promoter, which leads to an increased production of the enzyme, such as tyrosinase.

The typical vector is a plasmid having one or more marker genes for antibiotic resistance, an origin of replication, and a variety of useful restriction sites for cloning or subcloning restriction fragments. A large number of naturally occurring *Streptomyces* plasmids have been described, many of which are conjugally proficient. Two such isolates, SLP1.2 and pIJ101, have formed the basis of a series of useful plasmid vectors. Thompson, C.J. et al., *Gene 20,* 51 (1982).

The plasmids of the SLP1 family, of which SPL1.2 is the largest detected member, were discovered as autonomous replicons in *S. lividans* 66 after interspecific matings with *S. coelicolor* A3(2). The SPL1 replicon is integrated in the *S. coelicolor* genome but can be excised together with various lengths of neighboring DNA to become autonomous in *S. lividans.* The SPL1 plasmids exist stably at a copy number of 4-5 per chromosome in *S. lividans* and have a narrow host range.

The 8.9 kb plasmid pIJ101 was discovered in *S. lividans* ISP5434 (Kieser, T. et al., *Mol. Gen. Genet. 185,* 223 (1982)) but can be conjugally transferred to a wide variety of *Streptomyces* species. Derivatives (e.g. pIJ102) have been isolated from the plasmid which have similar properties but are smaller. Kieser, T. et al. (1982), *supra.* Plasmid pIJ101 has a copy number of 100-300 per chromosome equivalent in most hosts and a minimum replicon of less than 2.1 kb. Derivatives carrying drug-resistance determinants have been constructed to act as vectors, and a chimeric plasmid which can be used as shuttle vector between *E. coli* and *Streptomyces* is available.

The temperature phage φC31 has a wide host range within the streptomycetes and lysogenizes *S. coelicolor* A3(2) via a site-specific integration event. Lomovshaya, M. et al., *Bacteriol Rev. 44,* 206 (1980). Up to 42.4 kb of DNA can be packaged within a viable phage particle, but only 32 kb (at the most) of the DNA contains the genetic information essential for plaque formation. Derivatives of φC31 containing deletions can be used as vectors, and recombinant phages can either be grown lytically or used to lysogenize suitable streptomycete strains.

A limited number of genes cloned from various streptomycetes have been important in constructing vectors. The aminoglycoside phosphotransferase gene from *S. fradiae,* the aminoglycoside acetyltransferase gene (aac) from *S. fradiae,* and the ribosomal RNA methylase gene from *S. azureus,* that endows thiostrepton-resistance (tsr) have been used in pairwise combination to yield vectors allowing insertional inactivation. More recently, the tyrosinase gene, *mel,* whose product governs the synthesis of the brown pigment melanin from tyrosine, have been cloned from *S. antibioticus* and used to construct vectors that allow a visual recognition of recombinants. Katz, E. et al. (1983), *supra.*

The plasmid vector pIJ702 is useful for generalized cloning of DNA into a wide range of *Streptomyces,* allowing a visual recognition of transformant colonies containing recombinant plasmids. The vector contains a thiostrepton-resistance determinant, *tsr,* for genetic selection and a tyrosinase gene, *mel,* whose product directs the synthesis of the brown pigment, melanin, from tyrosine. Insertional inactivation of the *mel* gene leads to colorless transformants, whereas the vector yields dark brown colonies. Unique sites for *Bgl*II, *Sac*I and *Sph*I are present in the *mel* gene. The vector can also be used for cloning DNA fragments generated by *Kpn*I or *Pst*I, though without easy recognition of recombinants. Unique restriction sites for *Bam*HI and *Xho*I are not available for cloning, and insertion at the unique *Cla*I site inactivates the *tsr* gene, eliminating the genetic selection. The copy number of the vector is 40-300.

The vector is comprised of a 1.1 kb *Bcl*I fragment from *S. azureus,* containing the *tsr* gene (Thompson et al. (1982), *supra*), two contiguous *Bcl*I fragments, occupying 3.0 kb, from the *S. lividans* plasmid pIJ102 (Kieser, T. et al. (1982), *supra*), and a 1.55 kb *Bcl*I fragment from *S. antibioticus* containing the *mel* gene.

pBR322-derived plasmids are very common. They possess a pair of antibiotic resistance genes which confer antibiotic resistance when *Escherichia coli* are successfully transformed. Typically the insertion of a DNA segment is made so that one of the antibiotic resistance genes is inactivated. Selection then is accomplished by selecting for *E. coli* exhibiting antibiotic resistance conferred by the second gene. Bolivar, F. et al., *Gene 2,* 95 (1977); and Sutcliff, J., *Proc. Natl. Acad. Sci., USA 75,* 3737 (1978).

Another example of transforming vectors is the bacteriophage. The M13 series are modified filamentous *E. coli* bacteriophage containing single stranded circular DNA. The M13 series carry the *lacZ* gene for β-galactosidase and will metabolize the galactose analog Xgal to produce a blue color. Placing a cloned insert into the polylinker sequence located in the amino terminus of the *lacZ* gene inactivates the gene. Microorganisms carrying an M13 with an inactivated *lacZ* (representing a cloned insert) are distinguishable from those carrying an M13 with an active *lacZ* gene by their lack of blue color. Messing, J. et al., *Proc. Natl. Acad. Sci., USA 74,* 3642 (1977); and Messing, J., *Methods in Enzymology,* Vol 101, 20 (1983).

Other transforming vectors are the pUC series of plasmids. They contain the ampicillin resistance gene and origin of replication from pBR322, and a portion of the *lacZ* gene of *E. coli.* The *lac* region contains a polylinker sequence of restriction endonuclease recognition sites identical to those in the M13 series. The pUC series have the advantage that they can be amplified by chloramphenicol. When a DNA fragment is cloned into the *lac* region the lac gene is inactivated. When *E. coli* containing a pUC plasmid with an inactivated *lacZ* gene is grown in the presence of isopropylthiogalactoside (IPTG) and 5-bromo-4-chloro-3-indolyl β-D-galactopyranoside (Xgal) its colonies are white. If it carries a pUC plasmid with an active *lacZ* gene its colonies are blue. Vieira, J. et al., *Gene 19,* 259 (1982). Bacteria are transformed by means conventional in the art.

Transformation of Microorganism

The genus *Streptomyces* is one of three aerobic genera of bacteria of the order *Actinomycetales. Streptomyces* are Gram-positive, mycelial, spore-forming bacteria. Several naturally occurring *Streptomyces* plasmids have been described. *Streptomyces lividans TK64* has no tyrosinase gene and produces no melanin. The plasmid pIJ702 has the tyrosinase gene and is a high copy number plasmid. This results in at least a 3 times increase in tyrosinase production over strains having a tyrosinase gene on the chromosome. Applicants have used the plasmid pIJ702 to transform *Streptomyces lividans* TK64 to a high potential for production of extracellular melanin. Transformation is carried out by means standard in the art. Similarly the tyrosinase gene can be used to transform a variety of microorganisms after insertion into vectors which are useful for transforming the various host microorganisms.

Bluescript (obtained from Stratagene, LaJolla, CA) is a pUC derivative having a β-galactosidase color indicator and a lac promoter. Applicants have modified the Bluescript plasmid by inserting a tyrosinase gene. This modified plasmid was used to successfully transfer *E. coli* which formed pigmented colonies.

Mutagenesis

Mutations can be induced in microorganisms which are capable of producing melanins. The mutations can lead to a reduction, increase or no change in the production of melanins. Mutations are selected which lead to the enhanced production of melanins. The mutations can be induced by techniques known in the art which include radiation, such as ultraviolet light or gamma-radiations, as well as chemical mutagens such as ethidium bromide and ethyl methane sulfonate.

## C. Extraction of Melanin

Melanin has been extracted from bacterial cells with 0.5N NaOH at room temperature and at 100°C. Pigmented fractions were found to be: (1) soluble in acid and base; (2) soluble in ethyl alcohol and base; and (3) soluble base only. Pavlenko, G.V. et al. (1981), *supra.*

Soluble melanin can now be extracted from the medium and purified. This can be done by first removing cells and particulate matter such as filtration or centrifugation. If filtration is used, then a variety of filtration methods are known in the art including filtration through glass wool. If centrifugation is used, then 5,000 X gravity is usually sufficient. The melanin is then precipitated at about pH 0.5 to about 2.5, preferably about 1.5. Precipitated melanin is removed by either filtration or centrifugation. The melanin is washed by successive resolubilization at high pH, i.e. about pH 7.0 to about pH 9.0, preferably about pH 8.0, and precipitation at low pH followed by filtration or centrifugation. The melanin may also be concentrated using molecular weight filtration, such as reverse osmosis.

The invention is further illustrated by the following non-limiting examples.

## EXAMPLE 1

### Extractions of Melanin

Melanin produced in the following examples was extracted from the growth medium by the following procedure.

Cultures were filtered through glasswool to remove mycelium. Alternatively, particulate matter and cells were removed from the growth medium by centrifugation at 5,000 X gravity. The pH of the melanin containing medium was then reduced to about 1.5 with HCl. The precipitated melanin was removed by centrifugation at 6,800 X gravity. The precipitate was then removed and resolubilized at pH 8.0. The resolubilized melanin was washed by doubling the value of the liquid with sterile distilled $H_2O$. The process of precipitation, removal, resolubilization and washing is repeated 4 times in order to substantially remove any non-precipitable impurities. The product may be dried to completion in an oven at 200°C for 48 hours, if desired.

## EXAMPLE 2

### Prior Art Melanin Production

Example 2 is set forth to show the prior art production of melanin. The method is taken from Hopwood, D.A. et al., "Genetic Manipulation of Streptomyces: A Laboratory Manual" The John Innes Foundation (1985).

Melanin production by *Streptomyces lividans* TK64 (pIJ702).

### Preparation of Growth Medium

MMT MEDIUM was prepared from the following ingredients as described below.

### MM MEDIUM:

| | |
|---|---|
| L-asparagine | 0.5 g |
| $X_2HPO_4$ | 0.5 g |
| $MgSO_4.7H_2O$ | 0.2 g |
| $FeSO_4.7H_2O$ | 0.01 g |
| $H_2O$ | 1000 ml |

The ingredients were dissolved in water, adjusted to pH 7.0-7.2 with NaOH, 100 ml placed into 500 ml flasks, and autoclaved for 20 minutes.

The following sterile stocks were prepared:

*Difco Casaminoacids (30%) (50x Stock)

*Glucose (50%) (50x Stock)

*$CuSO_4$.5 $H_2O$ (0.50%) (1000x Stock)

*Tyrosinase Inducer:

| L-methionine (1%)<br>L-tyrosine (3%)<br>L-leucine (5%) | (33.3x Stock) |
|---|---|

*Tiger Milk:

| L-arginine (0.75%)<br>L-cystine (0.75%)<br>L-histidine (1.0%)<br>DL-homoserine (0.75%) | (133.3x Stock) |
|---|---|
| L-phenylalanine (0.75%)<br>L-proline (0.75%)<br>adanine (0.15%)<br>uracil (0.15%) | Does not dissolve completely forms a white, milk-like solution |
| nicotinamide (0.01%)<br>thiamine (0.01%) | |

The following ingredients were combined to prepare MMT medium:

100 ml MM MEDIUM

2 ml Casaminoacids

2 ml Glucose

750 ul Tiger Milk

For tyrosine and melanin production, the following ingredients were also included:

100 ul $CuSO_4$.5 $H_2O$

3 ml Tyrosinase Inducer

### Inoculation and Growth of TK64 (pIJ702)

A small amount of the bacteria were scraped from the top of the plate and transferred into 10 ml of sterile water which was mixed and pipeted into six-500 ml flasks containing 100 ml of MMT. Cultures were grown at 30°C, and 120 RPM for 3 days.

### Results

Melanin was extracted as described in Example 1. The yield of melanin was about 100 mg/l, dry weight.

### EXAMPLE 3

### Enhanced Melanin Production by Modification of the Nitrogen Source

### Preparation of Growth Medium

The MMT medium of Example 2 was modified to contain 0.5 g/l $K_2HPO_4$, 0.2 g/l $MgSO_4$ $H_2O$, 0.01g/l $FeSO_4$, 8 g/l casein hydrolysate, 0.3 g/l tyrosine and 0.1 g/l methionine.

* All of these stocks were autoclaved prior to making the medium.

### Inoculation and Growth of TK64 (pIJ702)

11 liters of the modified MMT medium was inoculated with $1.4 \times 10^4$ spores/ml *S. lividans* TK64(pIJ702) in 1 liter flasks containing 333 ml of growth medium. Cultures were grown for 3 days at 31°C with shaking at 150 RPM.

### Results

Melanin was extracted as described in Example 1. A total of 17.44 grams wet weight of melanin was obtained from 11 liters of medium. This was an average of 0.158 grams per liter dry weight. The yield ranged from 100-300 mg/l dry weight

### Example 4

Enhanced Melanin Production by Removal of Carbohydrate Inhibitor

### Preparation of Growth Medium

The medium preparation of Example 3 was repeated except that glucose was removed as a carbon source.

### Inoculation and Growth of TK64 (pIJ702)

250 ml of medium in a 1 liter flask was inoculated with $5.8 \times 10^3$ spores/ml of *S. lividans* TK64 pIJ702. Cultures were grown at 30°C, with shaking at 170 RPM for 3 days.

### Results

Melanin was extracted as described in Example 1. 3.569 grams wet weight of melanin was obtained from 250 ml of medium. This was 14.28 grams wet weight of melanin per liter of medium. The average dry weight of melanin produced was about 1 gram per liter. Melanin production ranged from 0.7 to 1.4 grams per liter, dry weight.

### EXAMPLE 5

Enhancement of Melanin Production With Casein Peptone Medium

### Preparation of Growth Medium

The medium preparation of Example 4 was repeated except that casein hydrolysate (obtained from Sigma Chemical) was replaced by an equivalent amount of casein peptone.

### Inoculation and Growth of TK64 (pIJ702)

Each 1 liter flask containing 250 ml of culture medium was inoculated with $5.8 \times 10^3$ spores/ml *S. lividans* TK64 (pIJ702). Cultures were incubated for 90 hours, at 30°C with shaking at 150 RPM.

### Results

Melanin was extracted as described in Example 1. Melanin was obtained at an average yield of 2.2 grams per liter, dry weight. The range of melanin production was between 1.8 and 2.6 grams per liter, dry weight.

EXAMPLE 6

Enhancement of Melanin Productioni With Tyrosine

Preparation of Growth Medium

The medium preparation of Example 5 was repeated except that the quantity of casein hydrolysate was replaced by casein peptone varying quantities of tyrosine were added. In different flasks the concentration of tyrosine in grams per liter was 0.3, 0.6, 0.9, 1.2 and 1.5.

Inoculation and Growth of TK64 (pIJ702)

Each 1 liter flask containing 250 ml of culture medium was inoculated with 5.8x10$^3$ spores/ml of *S. lividans* TK64 (pIJ702). Cultures were incubated for 90.5 hours, at 30°C with shaking at 150 RPM.

Results

The optical density at 400 nm ($OD_{400}$) of cultures medium in the flasks was read at intervals. The average optical density after 90.5 hours of incubation was:

| Tyrosine in Grams/liter | $OD_{400}$ |
|---|---|
| 0.3 | 0.621 |
| 0.6 | 1.009 |
| 0.9 | 1.354 |
| 1.2 | 1.520 |
| 1.5 | 1.523 |

The melanin was extracted as described in Example 1. The yield of melanin was 26 gram wet weight per liter of medium in the flask in which the tyrosine concentration was 1.5 g/l. This is 3.9 g/l dry weight.

Example 7

Production of Melanin In a Bioreactor

Preparation of Growth Medium

The growth medium was prepared as in Example 5. The medium contains 1.5 grams per liter of tyrosine. This medium contains no glucose or other carbon source except amino acids.

Inoculation and Growth of TK64 (pIJ702)

Spore stock of *S. lividans* TK64 (pIJ702) was diluted 1:10 in water. A starter culture was produced by adding 50 μl of dilute spore stock to 250 ml of culture medium in a 1 liter flask. The starter culture was incubated at 30°C with shaking until it reached mid-log phase.

Starter culture was then transferred to a 30 liter fermentor containing 20 liters of growth medium. Incubation was at 30°C with constant mixing at 225 RPM until the optical density of the medium reached about 1.3 at 400 nm ($OD_{400}$). Aeration during fermentation was by constant air flow at 1 liter of air per minute for 40 hours, and by 2.5 liters per minute for 40-60 hours, then by 3.0 liters per minute for the remaining 60-120 hours.

Results

Melanin was extracted as described in Example 1. The yield of melanin was about 1.7 grams per liter.

EXAMPLE 8

Production of Melanin In A Bioreactor

Preparation of Growth Medium

The growth medium was prepared as in Example 5. The medium contains 1.5 grams per liter of tyrosine. This medium contains no glucose or other carbon source except amino acids.

Inoculation and Growth of TK64 (pIJ702)

Spore stock of *S. lividans* TK64 (pIJ702) was diluted 1:10 in water. A starter culture was produced by adding 50 $\mu$l of dilute spore stock to 250 ml of culture medium in a 1 liter flask. The starter culture was incubated at 30°C with shaking until it reached mid-log phase. Starter culture was then transferred to a 42 liter fermentor containing 35 liters of growth medium. Incubation was at 30°C with constant mixing at 225 RPM until the optical density of the medium reached about 1.3 at 400 nm ($OD_{400}$). Aeration was by constant airflow at 1.5 liters of air per minute for 36 hours, 4.0 liters per minutes for 36-48 hours, and 5.0 liters per minute for the final 48-120 hours. Antifoam was added daily after 48 hours.

Results

Melanin was extracted as described in Example 1. The yield of melanin was about 2.0 grams per liter.

According to preferred embodiments in the claimed method of producing melanins the growth medium contains casein hydrolysate or casein peptone in combination with tyrosine and lacks glucose. Furthermore the microorganism is preferably transformed by a vector which contains one or more DNA sequences coding for an enzyme useful in the production of melanins said enzymes being preferably tyrosinase, laccase or phenyloxidase. When using a Streoptomyces or Escherichia bacterium said enzyme is preferably tyrosinase.

Thus a preferred growth medium is a medium comprising at least about 0,2 g dry weight of melanins per liter of growth medium, said melanins having been produced in said growth medium by a microorganism grown in said growth medium. Especially such a growth medium comprises at least about 1 g dry weight of melanins per liter and more preferably at least about 1,5 g and especially at least about 2 g dry weight of melanins per liter growth medium.

In an especially preferred method of producing melanins, comprising (a) selecting a DNA sequence coding for an enzyme useful in the production of melanins; (b) inserting at least one of said DNA sequence into a vector 3' to a host promoter; (c) tranforming a host bacterium with said vector; (d) growing said transformed bacterium, in a suitable growth medium until the concentration of melanins in the medium is at least about 0,2 g dry weight per liter; and (e) extracting melanin from said growth medium, the host is preferably Streptomyces, and said Streptomyces is preferably selected from the group consisting of Streptomyces lividans, Streptomyces scabies, Streptomyces antibioticus, Streptomyces coelicolor, Streptomyces fradiae and Streptomyces azureus. Also in this especially preferred method the growth medium preferably contains casein hydrolysate or casein peptone in combination with tyrosine and lacks glucose and the enzyme is preferably tyrosine, laccase or phenyloxidase. Preferably also in this method the melanins are extracted from the growth medium by acid precipitation or by reverse osmosis.

In an alternative preferred method of producing melanins comprising (a) growing a Streptomyces bacterium containing a gene coding for an enzyme useful in the production of melanins in a growth medium (i) containing tyrosine and a tyrosine containing substance and (ii) lacking glucose under attenuated fermentation conditions until the concentration of melanins in the medium is at least about 0,2 grams dry weight per liter; and (b) extracting said melanins from the growth medium, said tyrosine containing substrate is preferably casein hydrolysate or casein peptone, said Streptomyces is preferably Streptomyces lividans, especialls S. lividans TK64 containing the vector pIJ702. In this method growing is preferably conducted in a bioreactor.

In the shown methods of producing melanins, especially the last mentioned preferred method, said melanins are preferably extracted by (i) removing cells and other particulates from the growth medium; (ii) precipitating said melanins at about pH 1,5; and (iii) resolubilizing said melanins at about pH 8,0. Preferably said precipitating and resulubilizing are repeated two to four times.

In the culture which is used is a means for conducting the method for producing melanins and which comprises a growth medium and a microoranism capable of producing melanins, said culture being capable of producing at least about 0,2 g preferably at least 2 grams dry weight of melanins per liter the growth medium preferably contains (a) tyrosine and a tyrosine-containing substrate and (b) lacks glucose and preferably the tyrosine-containing substrate is casein hydrolysate or casein peptone, the microorganism preferably is Streptomyces or Escherichia, especially Streptomyces lividans, most preferably S. lividans TK64.

While the invention has been disclosed by reference to the details of preferred embodiments, the disclosure is intended in an illustrative rather than in a limiting sense, as it is contemplated that modifications will readily occur to those skilled in the art, within the spirit of the invention and the scope of the appended claims.

## Claims

1. A method of producing melanins, comprising:

   (a) inoculating a melanin producing microorganism in a suitable growth medium;

   (b) growing said microorganism under fermentation conditions comprising (i) a supply of dissolved oxygen around the rates of 20 - 90 %; (ii) a temperature range at 26°C to 60°C and (iii) a pH range between 6,8 to 7,2 until the concentration of extracellular melanins in the medium is at least about 0.2 grams dry weight per liter; and

   (c) extracting said extracellular melanins from the growth medium.

2. The method of claim 1 wherein the microorganism is a bacterium or a fungus.

3. The method of claim 1 wherein the growth medium lacks glucose.

4. The method of claim 1 wherein the growth medium contains a tyrosine containing substance selected from casein hydrolysate or casein peptone.

5. The method of one or more of claims 1 to 4 wherein the growth medium contains tyrosine, especially in an amount of 1.0 - 1.5 g/l.

6. The method of claim 1 wherein melanins are extracted from the growth medium by acid precipitation or by reverse osmosis.

7. The method of claim 1 wherein the microorganism is transformed by a vector which contains one or more DNA sequences coding for an enzyme useful in the production of melanins.

8. A method of producing melanins, comprising:

   (a) selecting a DNA sequence coding for an enzyme useful in the production of melanins;

   (b) inserting at least one of said DNA sequence into a vector 3' to a host promoter;

   (c) transforming a host bacterium with said vector;

   (d) growing said transformed bacterium, in a growth medium under fermentation conditions comprising (i) a supply of dissolved oxygen around the rates of 20 - 90 %; (ii) a temperature range at 26°C to 60°C and (iii) a pH range between 6,8 to 7,2 until the concentration of melanins in the medium is at least about 0.2 grams dry weight per liter; and

   (e) extracting extracellular melanin from said growth medium.

9. The method of claim 8, wherein said DNA sequence codes for tyrosinase and is obtained from a fungus, a bacterium, a human, an animal or a plant.

10. The method of claim 8, wherein said host is Streptomyces.

11. The method of claim 8, wherein the vector is a plasmid or bacteriophage derived from SLP1, pIJH101, øC31, pIJ702, M13 or pUC.

12. A method for producing melanins, comprising:

    (a) growing a Streptomyces bacterium containing a gene coding for an enzyme useful in the production of melanins in a growth medium (I) containing tyrosine and a tyrosine containing substance and (II) lacking glucose under fermentation conditions comprising (i) a supply of dissolved

EP 0 363 792 B1

oxygen around the rates of 20 - 90 %; (ii) a temperature range at 26°C to 60°C and (iii) a pH range between 6,8 to 7,2 until the concentration of melanins in the medium is at least 0.2 grams dry weight per liter; and
(b) extracting said extracellular melanins from the growth medium.

**13.** The method of claim 12 wherein said Streptomyces is S. lividans TK64 (ATCC 35287) containing the vector pIJ702.

**14.** A culture comprising a growth medium, a microorganism capable of producing melanin and at least 0.2 grams dry weight of melanin per liter wherein said melanin is produced in said growth medium by said microorganism wherein the procedure of growing a melanin-producing microorganism is performed under fermentation conditions comprising (i) a supply of dissolved oxygen around the rates of 20 - 90 %; (ii) a temperature range at 26°C to 60°C and (iii) a pH range between 6,8 to 7,2 until the concentration of melanin in the medium is at least 0.2 grams dry weight per liter of growth medium.

**15.** A culture according to claim 14 wherein the microorganism capable of producing melanin is Streptomyces.

**16.** A method of producing melanins, comprising:
(a) inoculating a melanin producing microorganism in a suitable growth medium (i) containing tyrosine and a tyrosine containing substance and (ii) lacking glucose; and
(b) growing said microorganism until the concentration of extracellular melanins in the medium is at least about 0.2 grams dry weight per liter; and
(c) extracting said extracellular melanins from the growth medium.

**17.** A method of producing melanins, comprising:
(a) selecting a DNA sequence coding for an enzyme useful in the production of melanins:
(b) inserting at least one of said DNA sequence into a vector 3' to a host promoter;
(c) transforming a host bacterium with said vector;
(d) growing said transformed bacterium in a growth medium (i) containing tyrosine and a tyrosine containing substance and (ii) lacking glucose;
(e) growing said microorganism until the concentration of extracellular melanins in the medium is at least about 0.2 grams dry weight per liter; and
(f) extracting said extracellular melanins from the growth medium.

**18.** A method of producing melanins, comprising:
(a) growing a Streptomyces bacterium containing a gene coding for an enzyme useful in the production of melanins in a grwoth medium (i) containing tyrosine and a tyrosine containing substance and (ii) lacking glucose until the concentration of extracellular melanins in the medium is at least about 0.2 grams dry weight per liter; and
(b) extracting said extracellular melanins from the growth medium.

**Patentansprüche**

**1.** Verfahren zum Herstellen von Melaninen, umfassend:
(a) Inokulieren eines Melanin-produzierenden Mikroorganismus in einem geeigneten Züchtungsmedium;
(b) Züchten des Mikroorganismus unter Fermentationsbedingungen, umfassend
(i) ein Zuführen von gelöstem Sauerstoff mit Raten von rund 20-90%;
(ii) einen Temperaturbereich von 26°C bis 60°C und
(iii) einen pH-Bereich zwischen 6,8 und 7,2,
bis die Konzentration extrazellulärer Melanine im Medium mindestens etwa 0,2 Gramm Trockengewicht pro Liter ist; und
(c) Extrahieren der extrazellulären Melanine aus dem Züchtungsmedium.

**2.** Verfahren nach Anspruch 1, worin der Mikroorganismus ein Bakterium oder ein Pilz ist.

**3.** Verfahren nach Anspruch 1, worin es dem Züchtungsmedium an Glucose mangelt.

15

4. Verfahren nach Anspruch 1, worin das Züchtungsmedium eine Tyrosin-enthaltende Substanz enthält, die aus Caseinhydrolysat oder Caseinpepton gewählt ist.

5. Verfahren nach einem oder mehreren der Ansprüche 1 bis 4, worin das Züchtungsmedium Tyrosin, insbesondere in einer Menge von 1,0-1,5 g/l, enthält.

6. Verfahren nach Anspruch 1, worin Melanine durch Säurefällung oder durch Umkehrosmose aus dem Züchtungsmedium extrahiert werden.

7. Verfahren nach Anspruch 1, worin der Mikroorganismus mit einem Vektor transformiert ist, welcher eine oder mehrere DNA-Sequenzen enthält, welche ein Enzym kodieren, das bei der Produktion von Melaninen brauchbar ist.

8. Verfahren zur Herstellung von Melaninen, umfassend:

(a) Selektieren einer DNA-Sequenz, welche ein Enzym kodiert, das bei der Produktion von Melaninen brauchbar ist;
(b) Inserieren von mindestens einer dieser DNA-Sequenz in einen Vektor 3' zu einem Host-Promotor;
(c) Transformieren eines Host-Bakteriums mit dem Vektor;
(d) Züchten des transformierten Bakteriums in einem Züchtungsmedium unter Fermentationsbedingungen, umfassend

(i) ein Zuführen von gelöstem Sauerstoff mit Raten von rund 20-90%;
(ii) einen Temperaturbereich von 26°C bis 60°C und
(iii) einen pH-Bereich zwischen 6,8 und 7,2,

bis die Konzentration an Melaninen im Medium mindestens etwa 0,2 Gramm Trockengewicht pro Liter ist; und
(e) Extrahieren von extrazellulärem Melanin aus dem Züchtungsmedium.

9. Verfahren nach Anspruch 8, worin die DNA-Sequenz Tyrosinase kodiert und von einem Pilz, einem Bakterium, einem Menschen, einem Tier oder einer Pflanze erhalten wird.

10. Verfahren nach Anspruch 8, worin der Host Streptomyces ist.

11. Verfahren nach Anspruch 8, worin der Vektor ein Plasmid oder ein Bakteriophage ist, der von SLP1, pIJH101, øC31, pIJ702, M13 oder pUC abgeleitet ist.

12. Verfahren zur Herstellung von Melaninen, umfassend

(a) Züchten eines Streptomyces-Bakteriums, welches ein Gen enthält, das ein Enzym kodiert, welches bei der Produktion von Melaninen in einem Züchtungsmedium brauchbar ist, welches (I) Tyrosin und eine Tyrosin-enthaltende Substanz enthält, und (II) welchem es an Glucose mangelt, unter Fermentationsbedingungen, umfassend

(i) ein Zuführen von gelöstem Sauerstoff mit Raten von rund 20-90%;
(ii) einen Temperaturbereich von 26°C bis 60°C und
(iii) einen pH-Bereich zwischen 6,8 und 7,2,

bis die Konzentration an Melaninen im Medium mindestens 0,2 Gramm Trockengewicht pro Liter ist; und
(b) Extrahieren der extrazellulären Melanine aus dem Züchtungsmedium.

13. Verfahren nach Anspruch 12, worin der Streptomyces S. lividans TK64 (ATCC 35287), welcher den Vektor pIJ702 enthält, ist.

14. Kultur, umfassend ein Züchtungsmedium, einen Mikroorganismus, der Melanin produzieren kann, und mindestens 0,2 Gramm Trockengewicht Melanin pro Liter, worin das Melanin in dem Züchtungsmedium vom Mikroorganismus produziert wird, wobei das Verfahren zur Züchtung eines Melanin-produzierenden Mikroorganismus unter Fermentationsbedingungen durchgeführt wird, welche umfassen:

(i) ein Zuführen von gelöstem Sauerstoff mit Raten von rund 20-90%;
(ii) einen Temperaturbereich von 26°C bis 60°C und

(iii) einen pH-Bereich zwischen 6,8 und 7,2,
bis die Konzentration an Melanin im Medium mindestens 0,2 Gramm Trockengewicht pro Liter Wachstumsmedium ist.

15. Kultur nach Anspruch 14, worin der Mikroorganismus, welcher Melanin produzieren kann, Streptomyces ist.

16. Verfahren zur Herstellung von Melaninen, umfassend:
(a) Inokulieren eines Melanin-produzierenden Mikroorganismus in einem geeigneten Züchtungsmedium, welches (i) Tyrosin und eine Tyrosin-enthaltende Substanz enthält, und welchem es (ii) an Glucose mangelt; und
(b) Züchten des Mikroorganismus bis die Konzentration extrazellulärer Melanine im Medium mindestens etwa 0,2 Gramm Trockengewicht pro Liter ist; und
(c) Extrahieren der extrazellulären Melanine aus dem Züchtungsmedium.

17. Verfahren zur Herstellung von Melaninen, umfassend:
(a) Selektieren einer DNA-Sequenz, welche ein Enzym kodiert, das bei der Produktion von Melaninen brauchbar ist;
(b) Inserieren von mindestens einer dieser DNA-Sequenz in einen Vektor 3' zu einem Host-Promotor;
(c) Transformieren eines Host-Bakteriums mit dem Vektor;
(d) Züchten des transformierten Bakteriums in einem Wachstumsmedium, welches (i) Tyrosin und eine Tyrosin-enthaltende Substanz enthält, und welchem es (ii) an Glucose mangelt;
(e) Züchten des Mikroorganismus bis die Konzentration extrazellulärer Melanine im Medium mindestens etwa 0,2 Gramm Trockengewicht pro Liter ist; und
(f) Extrahieren der extrazellulären Melanine aus dem Züchtungsmedium.

18. Verfahren zur Herstellung von Melaninen, umfassend:
(a) Züchten eines Streptomyces-Bakteriums, welches ein Gen enthält, das ein Enzym kodiert, welches bei der Produktion von Melaninen in einem Züchtungsmedium brauchbar ist, welches (i) Tyrosin und eine Tyrosin-enthaltende Substanz enthält, und welchem es (ii) an Glucose mangelt, bis die Konzentration extrazellulärer Melanine im Medium mindestens etwa 0,2 Gramm Trockengewicht pro Liter ist; und
(b) Extrahieren der extrazellulären Melanine aus den, Wachstumsmedium.

**Revendications**

1. Procédé de production de mélanines, comprenant les étapes consistant à :
(a) inoculer un micro-organisme produisant de la mélanine dans un milieu de croissance approprié ;
(b) cultiver ledit micro-organisme dans des conditions de fermentation comprenant (i) un apport d'oxygène dissous aux taux d'environ 20 à 90 %, (ii) une température comprise entre 26°C et 60°C et (iii) un pH compris entre 6,8 et 7,2, jusqu'à ce que la concentration des mélanines extracellulaires dans le milieu soit d'au moins environ 0,2 gramme, en poids net, par litre ; et
(c) extraire lesdites mélanines extracellulaires du milieu de croissance.

2. Procédé selon la revendication 1, dans lequel le micro-organisme est une bactérie ou un champignon.

3. Procédé selon la revendication 1, dans lequel le milieu de croissance ne contient pas de glucose.

4. Procédé selon la revendication 1, dans lequel le milieu de croissance contient une substance contenant de la tyrosine, qui est choisie parmi un hydrolysat de caséine et la caséine-peptone.

5. Procédé selon une ou plusieurs des revendications 1 à 4, dans lequel le milieu de croissance contient de la tyrosine, en particulier en une quantité de 1,0 à 1,5 g/l.

6. Procédé selon la revendication 1, dans lequel on extrait les mélanines du milieu de croissance par précipitation avec un acide ou par osmose inverse.

17

**7.** Procédé selon la revendication 1, dans lequel le micro-organisme est transformé par un vecteur qui contient une ou plusieurs séquences d'ADN codantes pour une enzyme qu'on peut utiliser pour la production de mélanines.

**8.** Procédé de production de mélanines, comprenant les étapes consistant à :

(a) choisir une séquence d'ADN codante pour une enzyme qu'on peut utiliser pour la production de mélanines ;

(b) insérer au moins l'une desdites séquences d'ADN dans un vecteur en 3' sur un vecteur hôte ;

(c) transformer une bactérie hôte avec ledit vecteur ;

(d) cultiver ladite bactérie transformée, dans un milieu de croissance, dans des conditions de fermentation comprenant (i) un apport d'oxygène dissous aux taux d'environ 20 à 90 %, (ii) une température comprise entre 26°C et 60°C et (iii) un pH compris entre 6,8 et 7,2, jusqu'à ce que la concentration des mélanines dans le milieu soit d'au moins environ 0,2 gramme, en poids net, par litre ; et

(e) extraire la mélanine extracellulaire dudit milieu de croissance.

**9.** Procédé selon la revendication 8, dans lequel ladite séquence d'ADN est codante pour la tyrosinase et obtenue à partir d'un champignon, d'une bactérie, d'un être humain, d'un animal ou d'une plante.

**10.** Procédé selon la revendication 8, dans lequel ledit hôte est un *Streptomyces*.

**11.** Procédé selon la revendication 8, dans lequel le vecteur est un plasmide ou un bactériophage provenant de SLP1, pIJH101, øC31, pIJ702, M13 ou pUC.

**12.** Procédé de production de mélanines, comprenant les étapes consistant à :

(a) cultiver une bactérie du type *Streptomyces* contenant un gène codant pour une enzyme qu'on peut utiliser pour la production de mélanines, dans un milieu de croissance qui (I) contient de la tyrosine et une substance contenant de la tyrosine et (II) ne contient pas de glucose, dans des conditions de fermentation comprenant (i) un apport d'oxygène dissous aux taux d'environ 20 à 90 %, (ii) une température comprise entre 26°C et 60°C et (iii) un pH compris entre 6,8 et 7,2, jusqu'à ce que la concentration des mélanines dans le milieu soit d au moins 0,2 gramme, en poids net, par litre ; et

(b) extraire lesdites mélanines extracellulaires dudit milieu de croissance.

**13.** Procédé selon la revendication 12, dans lequel ledit *Streptomyces* est le *S. lividans* TK64 (ATCC 35287) contenant le vecteur pIJ702.

**14.** Culture comprenant un milieu de croissance, un micro-organisme capable de produire de la mélanine, et au moins 0,2 gramme, en poids net, de mélanine par litre, dans laquelle ladite mélanine est produite dans ledit milieu de croissance au moyen dudit micro-organisme, où le processus de croissance d'un micro-organisme producteur de mélanine est effectué dans des conditions de fermentation comprenant (i) un apport d'oxygène dissous aux taux d'environ 20 à 90 %, (ii) une température comprise entre 26°C et 60°C et (iii) un pH compris entre 6,8 et 7,2, jusqu'à ce que la concentration de mélanine dans le milieu soit d'au moins 0,2 gramme, en poids net, par litre de milieu de croissance.

**15.** Culture selon la revendication 14, dans laquelle le micro-organisme capable de produire la mélanine est un *Streptomyces*.

**16.** Procédé de production de mélanines, comprenant les étapes consistant à :

(a) inoculer un micro-organisme producteur de mélanine dans un milieu de croissance approprié qui (i) contient de la tyrosine et une substance contenant de la tyrosine et (ii) ne contient pas de glucose;

(b) cultiver ledit micro-organisme jusqu'à ce que la concentration des mélanines extracellulaires dans le milieu soit d'au moins environ 0,2 gramme, en poids net, par litre ; et

(c) extraire lesdites mélanines extracellulaires du milieu de croissance.

**17.** Procédé de production de mélanines, comprenant les étapes consistant à :

(a) choisir une séquence d'ADN codante pour une enzyme qu'on peut utiliser pour la production de mélanines ;

(b) insérer au moins une desdites séquences d'ADN dans un vecteur en 3' sur un promoteur hôte ;

(c) transformer une bactérie hôte avec ledit vecteur ;

(d) cultiver ladite bactérie transformée dans un milieu de croissance qui (i) contient de la tyrosine et une substance contenant de la tyrosine et (ii) ne contient pas de glucose ;

(e) cultiver ledit micro-organisme jusqu'à ce que la concentration des mélanines extracellulaires dans le milieu soit d'au moins environ 0,2 gramme, en poids net, par litre ; et

(f) extraire lesdites mélanines extracellulaires du milieu de croissance.

**18.** Procédé de production de mélanines, comprenant les étapes consistant à :

(a) cultiver une bactérie du type *Streptomyces* contenant un gène codant pour une enzyme qu'on peut utiliser pour la production de mélanines, dans un milieu de croissance qui (i) contient de la tyrosine et une substance contenant de la tyrosine et (ii) ne contient pas de glucose, jusqu'à ce que la concentration des mélanines extracellulaires dans le milieu soit d'au moins environ 0,2 gramme, en poids net, par litre ; et

(b) extraire lesdites mélanines extracellulaires du milieu de croissance.